Europäisches Patentamt

European Patent Office

Office européen des brevets

(11) Veröffentlichungsnummer: **0 272 546**
**A1**

(12) **EUROPÄISCHE PATENTANMELDUNG**

(21) Anmeldenummer: **87118242.4**

(22) Anmeldetag: **09.12.87**

(51) Int. Cl.4: **A61B 6/03**

(30) Priorität: **22.12.86 DE 3643978**

(43) Veröffentlichungstag der Anmeldung:
**29.06.88 Patentblatt 88/26**

(84) Benannte Vertragsstaaten:
**DE FR**

(71) Anmelder: **Siemens Aktiengesellschaft Berlin
und München
Wittelsbacherplatz 2
D-8000 München 2(DE)**

(72) Erfinder: **Klingenbeck, Klaus, Dr.
Dannberger Weg 8
D-8521 Niederlindach(DE)**
Erfinder: **Rührnschopf, Ernst-Peter,
Dipl.-Math.
Friedrich-Bauer-Strasse 7
D-8520 Erlangen(DE)**
Erfinder: **Schwierz, Günter, Dr.
Rödlaser Strasse 9
D-8524 Neunkirchen(DE)**

(54) **Computertomograph.**

㉗ Bei einem Computertomographen der dritten Generation mit fächerförmigem Röntgenstrahlenbündel und einer Meßeinheit aus Röntgenstrahlenquelle (l) und aus einer Reihe von Einzeldetektoren (l2) bestehendem Strahlenempfänger (3) soll der nicht-lineare Teilvolumeneffekt weitgehend eliminiert werden. Dieser Effekt ist dadurch bedingt, daß Objektstrukturen nur partiell in die endlich dicke tomographische Schicht eintauchen.

Zur Eliminierung dieses Effekts ist entweder der Brennfleck (ll) oder der Strahlenempfänger (3) gegenüber der idealisiert gedachten tomographischen Ebene (l3) seitlich versetzt, so daß die durch den Brennfleck (ll) und den Strahlenempfänger (3) definierte Ebene bei der Abtastung des Aufnahmeobjekts eine Taumelbewegung ausführt.

FIG 1

## Computertomograph

Die Erfindung betrifft einen Computertomograph der 3. Generation mit einer Meßeinheit aus einer Röntgenstrahlenquelle und einem Strahlenempfänger, bei der die Röntgenstrahlenquelle ein fächerförmiges Röntgenstrahlenbündel aussendet, das auf dem aus einer Reihe von Einzeldetektoren bestehenden Strahlenempfänger auftrifft, von denen jeder ein der empfangenen Strahlenintensität entsprechendes elektrisches Signal bildet, bei dem Mittel zur Drehung der Meßeinheit um das Meßfeld zur Durchstrahlung des Meßfeldes unter verschiedenen Richtungen vorgesehen sind, bei dem eine Meßwertverarbeitungseinheit vorhanden ist, der die Ausgangssignale der Einzeldetektoren, die bei den verschiedenen Durchstrahlungsrichtungen erzielt werden, zugeführt werden und die daraus die Schwächungswerte in dem Meßfeld bestimmt, und bei dem eine Bildwiedergabevorrichtung zur bildlichen Wiedergabe der berechneten Schwächungswerte vorhanden ist.

Ein Computertomograph dieser Art ist in der Fig. I dargestellt. Er weist eine Meßeinheit aus einer Röntgenstrahlenquelle I, die ein fächerförmiges Röntgenstrahlenbündel 2 aussendet, und einen Strahlenempfänger 3 auf, welcher aus einer Reihe von Einzeldetektoren, z.B. aus 5I2 Einzeldetektoren besteht. Der zu untersuchende Patient 4 liegt auf einer Lagerstatt 5. Zur Abtastung des Patienten 4 wird die Meßeinheit I, 3 um ein Meßfeld 9, in dem der Patient 4 liegt, um 360° gedreht. Die Drehachse ist mit I0 bezeichnet. Dabei wird die Röntgenstrahlenquelle I, die von einem Röntgengenerator 6 gespeist wird, gepulst oder mit Dauerstrahlung betrieben. Bei vorbestimmten Winkelpositionen der Meßeinheit I, 3 werden Sätze von Daten erzeugt, die vom Strahlenempfänger 3 einem Rechner 7 zugeführt werden, welcher aus den erzeugten Datensätzen die Schwächungskoeffizienten vorbestimmter Bildpunkte berechnet und auf einem Sichtgerät 8 bildlich wiedergibt. Auf dem Sichtgerät 8 erscheint demgemäß ein Bild der durchstrahlten Schicht des Patienten 4.

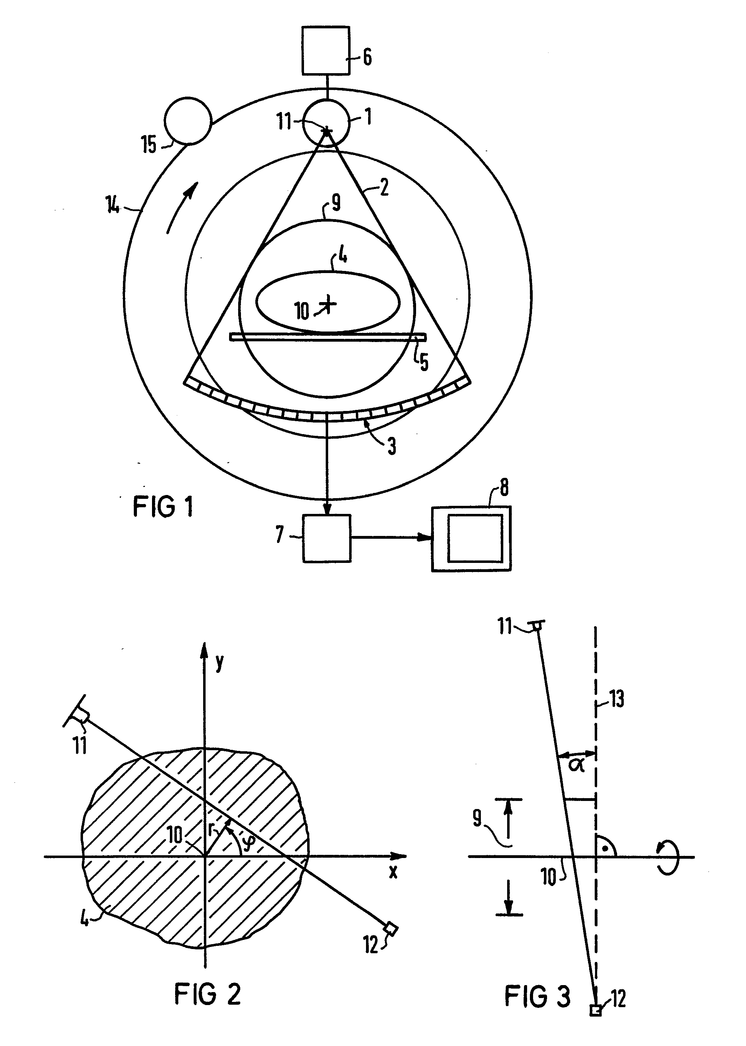

Mit Hilfe eines Computertomographen kann also die Verteilung des gewebeabhängigen Schwächungskoeffizienten $\mu$ in einer Transversalschicht des menschlichen Körpers bestimmt und bildlich dargestellt werden. In der Fig. 2 ist zur Erläuterung nur der Brennfleck II und ein Einzeldetektor I2 des Strahlenempfängers 3 dargestellt. Die für die Bildrekonstruktion erforderlichen Linienintegrale

$$p(r,\varphi) = \int_{L(r,\varphi)} \mu(x,y)\,ds$$

des Schwächungskoeffizienten längs des Strahlweges $L(r,\phi)$ sind der Messung wegen der erforderlichen endlichen Abmessung des Brennflecks II und der Einzeldetektoren I2 nur näherungsweise über die gemessenen Intensitäten

$$I(r,\phi) = I_0 \overline{\exp(-p(r,\phi))}$$

zugänglich. Der Querstrich bedeutet hier die Mittelung der in den Exponenten erhobenen Schwächungsintegrale über den Brennfleck und die Detektoreingangsfläche. $I_0$ ist die Strahlenintensität vor dem Objekt. Andere die Messung verfälschende Einflüsse, wie beispielsweise die endliche Breite des Röntgenenergiespektrums, werden hier nicht berücksichtigt.

Der durch Logarithmierung gewonnene Wert

$$\overline{p}(r,\phi) = -\ln(I(r,\phi)/I_0) = -\ln(\overline{\exp(-p(r,\phi))})$$

stellt nur im technisch unerreichbaren Grenzfall unendlich kleiner Detektor-und Brennfleckabmessungen den gesuchten Wert $p(r,\phi)$ richtig dar.

Tauchen Objektstrukturen nur partiell in die - bedingt durch die endlichen Höhenabmessungen von Einzeldetektor I2 und Brennfleck II - endlich dicke tomographische Schicht ein, so führt die Abweichung der Werte $\overline{p}$ von den Linienintegralen $p$ zu Bildstörungen, die die medizinische Diagnosefindung erschweren können. Wie eine Untersuchung gezeigt hat, sind beispielsweise die von Neuroradiologen bei Schädelaufnahmen häufig beklagten Artefakte in der hinteren Schädelgrube auf diesen Effekt zurückzuführen. Man faßt die genannten Bildstörungen unter dem Begriff "nicht-linearer Teilvolumeneffekt" (non linear partial volume effect) zusammen.

Bisher versucht man die Entstehung dieser Artefakte durch Wahl dünner tomographischer Schichten zu vermeiden. Dies hat mehrere Nachteile. Um die für die Diagnosefindung erforderliche Röntgendosis pro Schichtbild zu erzielen, müssen bei Verkleinerung der Abmessungen von Einzeldetektor und Brennfleck die Bildaufnahmezeit verlängert, sowie die Röntgenstrahlenquelle thermisch höher belastet werden, wodurch ihre Lebensdauer verkürzt wird. Da im Regelfall die Aufnahme einer Schicht unzureichend ist, sondern ein ganzes Objektvolumen durch Wahl eines hinreichend kleinen Schichtabstandes lückenlos dargestellt werden muß, führen dünne Schichten zur weiteren Verlängerung der gesamten Untersuchungszeit, zur zusätzlichen thermischen Belastung der Röntgenstrahlenquelle und zu einer Erhöhung der dem Patienten applizierten Röntgendosis.

Die Erfindung geht von der Erkenntnis aus, daß eine einzige nur partiell eintauchende Struktur Bildstörungen erzeugt, die auf den Ort der Struktur beschränkt sind, also keine Fernwirkungen zeigen. Sie äußern sich als minimale, vom Beobachter kaum wahrnehmbare Verfälschungen des berechneten lokalen Schwächungskoeffizienten und spielen für die Diagnosefindung keine Rolle. Artefakte entstehen nur dann, wenn mehrere örtlich verteilte Strukturen nur partiell eintauchen, typischerweise als dunkle, hell umsäumte Balken zwischen den Strukturen.

Der Erfindung liegt die Aufgabe zugrunde, einen Computertomographen der eingangs genannten Art so auszubilden, daß der nicht-lineare Teilvolumeneffekt durch einfache konstruktive Maßnahmen ohne Verlängerung der Bildaufnahmezeit sowie ohne höhere thermische Belastung der Röntgenstrahlenquelle weitgehend eliminiert wird.

Diese Aufgabe ist erfindungsgemäß dadurch gelöst, daß die durch den Strahlenempfänger und den Brennfleck der Röntgenstrahlenquelle definierte Ebene mit der Drehachse der Meßeinheit einen von 90° abweichenden Winkel bildet, so daß diese Ebene bei der Drehung der Meßeinheit eine Taumelbewegung ausführt.

Die Erfindung ist nachfolgend anhand eines in der Fig. 3 dargestellten Ausführungsbeispiels näher erläutert. In der Fig. 3 ist gezeigt, daß der Schwerpunkt des Brennflecks ll in geringem Abstand außerhalb der idealisiert gedachten tomographischen Ebene l3 angebracht ist. Es ist dies die von der Drehachse l0 senkrecht durchstoßene Ebene, in der die Schwerpunkte der Einzeldetektoren liegen. Dies führt zu einer taumelnden Bewegung der durch den Strahlenempfänger 3 und den Schwerpunkt des Brennflecks ll definierten Ebene bei der Drehung der Meßeinheit l, 3 um die Achse l0. Strukturpaare, die nur partiell in die ursprüngliche tomographische Ebene l3 eintauchen, werden durch diese Maßnahme teilweise entkoppelt, da sie sich nun häufiger als bei den bekannten Computertomographen nicht mehr gleichzeitig im Röntgenstrahlenbündel 2 befinden. Hierdurch nähert sich die Bildstörung dem lokalisierten, diagnostisch unbedeutsamen Störtyp, die Störbalken werden reduziert. Die Erfindung kann auch in der Weise realisiert werden, daß sich der Brennfleck ll in der Ebene l3 befindet, der Strahlenempfänger 3 mit den Einzeldetektoren aber gegenüber der Ebene l3 versetzt ist. Wesentlich ist also, daß die durch den Brennfleck ll und den Strahlenempfänger 3 definierte Ebene mit der Drehachse l0 einen von 90° abweichenden Winkel bildet. Der Winkel α, um den in Fig. 3 die Verbindungslinie zwischen dem Einzeldetektor l2 und dem Brennfleck ll gegenüber der Ebene l3 geneigt ist, beträgt zweckmäßigerweise mehr als 20 Prozent der kleinsten einstellbaren Schichtdicke.

In der Fig. l ist noch ein Drehkranz l4 dargestellt, auf dem die Meßeinheit l, 3 angeordnet ist, dessen Mittelpunkt mit der Drehachse l0 zusammenfällt und der durch einen Antriebsmotor l5 zur Drehung der Meßeinheit l, 3 angetrieben wird.

## Ansprüche

Computertomograph der 3. Generation mit einer Meßeinheit (l, 3) aus einer Röntgenstrahlenquelle (l) und einem Strahlenempfänger (3), bei der die Röntgenstrahlenquelle (l) ein fächerförmiges Röntgenstrahlenbündel (2) aussendet, das auf dem aus einer Reihe von Einzeldetektoren (l2) bestehenden Strahlenempfänger (3) auftrifft, von denen jeder ein der empfangenen Strahlenintensität entsprechendes elektrisches Signal bildet, bei dem Mittel (l4) zur Drehung der Meßanordnung (l, 3) um das Meßfeld (9) zur Durchstrahlung des Meßfeldes (9) unter verschiedenen Richtungen vorgesehen sind, bei dem eine Meßwertverarbeitungseinheit (7) vorhanden ist, der die Ausgangssignale der Einzeldetektoren (l2), die bei den verschiedenen Durchstrahlungsrichtungen erzielt werden, zugeführt werden und die daraus die Schwächungswerte vorbestimmter Punkte in der durchstrahlten Schicht des Aufnahmeobjektes (4) bestimmt und bei dem eine Bildwiedergabevorrichtung (8) zur bildlichen Wiedergabe der berechneten Schwächungswerte vorhanden ist, **dadurch gekennzeichnet,** daß die durch den Strahlenempfänger (3) und den Brennfleck (ll) der Röntgenstrahlenquelle (l) definierte Ebene mit der Drehachse (l0) der Meßeinheit (l, 3) einen von 90° abweichenden Winkel ( ) bildet, so daß diese Ebene bei der Drehung der Meßeinheit (l, 3) eine Taumelbewegung ausführt.

FIG 1

FIG 2

FIG 3

Europäisches
Patentamt

**EUROPÄISCHER RECHERCHENBERICHT**

Nummer der Anmeldung

EP 87 11 8242

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DE-A-2 729 833 (SIEMENS AG) <br> * Seite 8, Zeilen 11-21; Seite 9, Zeile 32 - Seite 10, Zeile 15; Figuren 1,6 * <br> --- | 1 | A 61 B 6/03 |
| X | FR-A-2 368 930 (SIEMENS AG) <br> * Seite 3, Zeilen 7-29; Seite 4, Zeilen 34-38; Seite 7, Zeilen 25-31; Figur 3 * <br> ----- | 1 | |
| | | | **RECHERCHIERTE SACHGEBIETE (Int. Cl.4)** <br><br> A 61 B |

Der vorliegende Recherchenbericht wurde für alle Patentansprüche erstellt

| Recherchenort | Abschlußdatum der Recherche | Prüfer |
|---|---|---|
| DEN HAAG | 19-02-1988 | RIEB K.D. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
..............................................................................................
& : Mitglied der gleichen Patentfamilie, übereinstimmendes
    Dokument

EPO FORM 1503 03.82 (P0403)